# EUROPÄISCHE PATENTSCHRIFT

(11) **EP 1 005 495 B1**
(45) Veröffentlichungstag und Bekanntmachung des Hinweises auf die Patenterteilung: **20.03.2002**
(21) Anmeldenummer: 98945281.8
(22) Anmeldetag: 24.08.1998
(51) Int. Cl.: C08B 11/193, C08B 15/06, B05D 1/20

(54) **AMINOALKYLTRIALKYLSILYLCELLULOSEN UND EIN VERFAHREN ZUR BESCHICHTUNG VON OBERFLÄCHEN**
AMINOALKYL TRIALKYL SILYL CELLULOSE AND A METHOD FOR COATING SURFACES
AMINOALKYL TRIALKYLSILYLCELLULOSES ET PROCEDE DE REVETEMENT DE SURFACES

(30) Priorität: 23.08.1997 DE 19736736
(43) Veröffentlichungstag der Anmeldung: 07.06.2000
(73) Patentinhaber: Molecular Machines & Industries GmbH, 69120 Heidelberg (DE)
(72) Erfinder: SEEGER, Stefan, D-93077 Bad Abbach (DE); LÖSCHER, Frank, D-93053 Regensburg (DE)
(74) Vertreter: Isenbruck, Günter, Dr.
(86) Internationale Anmeldenummer: EP9805365
(87) Internationale Veröffentlichungsnummer: WO9910383

(56) Entgegenhaltungen:
- EP-A- 0 562 378
- EP-A- 0 583 677
- PATENT ABSTRACTS OF JAPAN vol. 12, no. 155 (C-494), 12. Mai 1988 & JP 62 266170 A (NEC CORP), 18. November 1987 & DATABASE WPI Week 8815 Derwent Publications Ltd., London, GB; AN 100580
- PATENT ABSTRACTS OF JAPAN vol. 12, no. 155 (C-494), 12. Mai 1988 & JP 62 266167 A (NEC CORP), 18. November 1987 & DATABASE WPI Week 8815 Derwent Publications Ltd., London, GB; AN 100577 A

## Beschreibung

Die Erfindung betrifft neue Celluloseether, nämlich Aminoalkyltrialkylsilylcellulosen, deren Herstellung und deren Anwendung in einem Verfahren zur Beschichtung von Oberflächen mit definierten Molekülschichten.

Die Immobilisierung von Biomolekülen auf festen Trägern spielt bei einer Vielzahl moderner Analyse- und Trenntechniken, wie der Affinitätschromatographie, der Bioreaktortechnik und vor allem der analytischen Bio- und Chemosensorik eine entscheidende Rolle.

Zum Beispiel erfolgt ein Nachweis in Immuntests und Hybridisierungstests dadurch, daß an eine feste Substratoberfläche adsorbierte Rezeptomoleküle spezifisch mit den zu bestimmenden Spezies reagieren. Insbesondere im Bereich der hochempfindlichen Detektion einzelner DNA-, RNA-, Antigen- bzw. Proteinmoleküle, ist es von großer Bedeutung, daß die entsprechenden Moleküle spezifisch und fest an Oberflächen gebunden sind, und eine unspezifische Adsorption von Molekülen auf diesen Substratoberflächen unterbunden wird.

Es sind verschiedene Verfahren bekannt, um Biomoleküle auf festen Substratoberflächen zu binden.

Eine einfache Möglichkeit stellt eine adsorptive Immobilisierung dar, bei der durch nichtkovalente Wechselwirkungen eine rein adsorptive Bindung auf einer Substratoberfläche erfolgt. Diese Methode hat verschiedene Nachteile. Die Immobilisierung ist auf Substrate beschränkt, deren Oberflächenbeschaffenheit ein adsorptive Bindung zuläßt und eine ausreichende Stabilität gewährleistet. Durch unvollständige Beschichtung oder Desorptionsprozesse können Lücken in der Biomolekülschicht entstehen. Schließlich ist die Orientierung wie auch die Menge der Rezeptormoleküle unbefriedigend zu steuern, so daß eine reproduzierbare Herstellung nur schwierig zu erreichen ist.

Um diese Nachteile zu umgehen, wurde nach kovalenten Immobilisierungsverfahren gesucht, mit denen Biomoleküle über funktionelle Gruppen kovalent an feste Substrate gebunden werden können. Grundlage dieser kovalenten Verfahren sind bifunktionelle Brückenreagentien, die sowohl mit der Substratoberfläche als auch mit dem Biomolekül reagieren.

Diese häufig in mehreren Schritten verlaufenden Immobilisierungsverfahren sind sehr zeit- und materialaufwendig. Ein weiterer Nachteil dieser Methoden ist die Bildung von inhomogenen, polymeren Oberflächenstrukturen z.B. von in der Praxis erzeugten Silanfilmen in Gegenwart von Feuchtigkeit, siehe dazu z.B. Joachim Renken et al., Anal. Chem. 1996, 68, S. 176 bis 182 in "Multifrequency Evaluation of Different Immunosorbents on Acoustic Plate Mode Sensors".

Eine weitere Technik für die Kopplung von Biomolekülen auf festen Substratoberflächen ist die Self-Assembly (SA)-Technik (siehe z.B. Kevin L. Prime et al., J. Amer. Chem. Soc. 1993, 115, S. 10714 bis 10721 in "Adsorption of Proteins onto Surfaces Containing End-Attached Oligo(ethylenoxide): A Model System Using Self-Assembled Monolayers". Dabei bilden sich stabile Filme organischer Substanzen durch eine spontane Selbstanordnung der Moleküle bei der Adsorption auf festen Substraten. Die bekanntesten SA-Systeme sind organische Disulfide und Thiole auf Goldoberflächen. Ein Nachteil dieser Methode ist die Begrenzung auf wenige Substrattypen wie Metalle oder spezielle Oxide.

Verwandt mit der SA-Technik ist die Langmuir-Blodgett-(LB)-Technik. Spreitet man geeignete Substanzen auf eine Wasseroberfläche, so breiten sie sich zu einem monomolekularen Film aus. Mit dieser von Langmuir und Blodgett entwickelten Technik gelingt es, diese monomolekularen Filme auf feste Substrate zu übertragen (siehe Katharine B. Blodgett et al., Physical Review, Vol. 51, Juni 1937, S. 964 bis 982 in "Built-Up Films of Barium Stearate and Their Optical Properties"). Dieses Verfahren ist mit einem sehr geringen materiellen und zeitlichen Aufwand verbunden.

Besonders geordnete und stabile Filme erhält man durch den Einsatz sogenannter nicht-amphiphiler Stab-Haar-Polymere (z.B. M. Schaub et al., Thin Solid Films, 210/211, 1992, S. 397 bis 400 in "Investigation of molecular superstructures of hairy rodlike polymers by X-ray reflection).

Beim Übertrag auf das feste Substrat ergibt sich eine zur Tauchrichtung parallele Orientierung der Polymer-Stäbe. Eine besonders hohe Stabilität der Filme kann zusätzlich durch eine Quervernetzung von im Polymer vorhandenen Alkensubstituenten in einer [2+2]-Cycloaddition unter UV-Bestrahlung erreicht werden (Gerhard Wegner, Thin Solid Films, 216, 1992, S. 105 bis 116 in "Ultrathin Films of polymers: architecture, characterization and properties").

Mit besonderem Erfolg wurden Cellulosederivate eingesetzt, die olefinische Seitenketten besitzen. Diese können nach erfolgtem Filmübertrag auf das Substrat in einer modifizierten Lemieux-Oxidation in Carbonylgruppen umgewandelt werden, an welche Biomoleküle kovalent als "Schiffsche Basen" gekoppelt werden (WO-A 95/08770 oder Frank Löscher et al., Proc. SPIE Vol. 2928, 1996, S. 209 bis 219).

Durch Variation der Belichtungsdauer kann zusätzlich eine Variation der Belegungsdichte erreicht werden. An andere Cellulosederivate mit freien Amino- oder Hydroxylgruppen können Biomoleküle mittels eines bifünktionellen Brückenreagens wie Cyanurchlorid kovalent gekoppelt werden (siehe vorstehendes Zitat).

Nachteil dieses Verfahrens ist eine Begrenzung auf hydrophobe bzw. hydrophobisierte Substrattypen. So müssen beispielsweise hydrophile Glas- oder Quarzsubstrate vor einer Beschichtung mit hydrophoben LB-Substanzen in aufwendigen naßchemischen Schritten mit Hilfe von z.B. Silanderivaten hydrophobiert werden.

Daher ist es Aufgabe der Erfindung, ein Verfahren und eine dafür geeignete chemische Verbindung aufzufinden, womit unabhängig von der Hydrophilie von Oberflächen mindestens eine Molekülschicht auf möglichst verschiedenartige Oberflächen aufgebracht werden kann.

Eine Lösung dieser Aufgabe sind Polysaccharidderivate, enthaltend a) mindestens einen hydrophoben und b) mindestens einen ein Stickstoff enthaltenden Substituenten . Die erfindungsgemäßen Verbindungen sind dann dadurch gekennzeichnet, daß das Derivat ein Celluloseether ist, der als Substituenten a) eine Trialkylsilyl- unnd als Substituenten b) eine Aminoalkylgruppe aufweist, und einen Polymerisationsgrad von ≥5 hat. Dabei hat der Alkylrest insbesondere im Substituenten a) 1 oder 2 C-Atome und im Substituenten b) 2 bis 8 C-Atome . Zusätzlich kann das Polysaccharidderivat noch c) mindestens einen weiteren Substituenten enthalten, der eine photochemisch, radikalisch oder thermisch vernetzbare Gruppe trägt.

Unter den (gemischten) Celluloseethern sind vorliegend solche Verbindungen zu verstehen, bei denen einzelne der OH-Gruppen des Cellulosegrundgerüstes am H durch organische oder Organosilylgruppen ersetzt sind, d.h. das direkt dem O benachbarte Atom ein C oder Si ist. Darüber hinaus können unter dieser Bezeichnung aber auch noch Derivate verstanden werden, die zusätzlich weitere Substituenten (insbesondere am O der OH-Gruppe) tragen, beispielsweise ist der Substituent c) ein solcher. In den konkreten Molekülen (siehe dazu als Informationsbasis Lothar Brandt in Ullmann's Encyclopedia of Industrial Chemistry, Vol. A5, 2. Auflage, Stichwort "Cellulose Ethers", S. 461 ff.) muß nicht jede einzelne Moleküleinheit (Anhydroglucose-Einheit) im Celluloseethermolekül an einer oder mehreren OH-Gruppen substituiert sein, sondern die Verbindungsbezeichnung bezieht sich auf die Gesamtheit der Moleküle bzw. Moleküleinheiten, stellt also eine Mittelwertbezeichnung dar; im allgemeinen sind maximal 3 OH-Gruppen pro Moleküleinheit substituierbar. Zur Herstellung bzw. zum Verhalten von die Substituenten a) oder c) (jedoch nicht b)) enthaltenden Celluloseethern wird auf die vorstehende Literaturstelle Frank Löscher et al. und auf Dieter Klemm et al., Z. Chem., 24. Jg. (1984), Heft 2, S. 62 in "4-Dimethylamino-pyridin-katalysierte Synthese von Celluloseestern über organlösliche Synthese von Celluloseestern über organolösliche Trimethylcellulose" verwiesen.

Ein weitere Lösung der gestellten Aufgabe geht aus von dem bekannten Verfahren zur Immobilisierung von Biomolekülen auf einem beschichteten flächigen Trägermaterial, bei dem die Biomoleküle an oder in der Beschichtung gebunden werden. Das erfindungsgemäße Verfahren ist dann dadurch gekennzeichnet, daß das beschichtete flächige Trägermaterial innerhalb oder außerhalb der Beschichtung mindestens einen der vorstehenden (gemischten) Celluloseether enthält.

Im folgenden werden bevorzugt Ausführungsformen dieses Verfahrens näher erläutert.

Auf einer festen Oberfläche wird mindestens eine monomolekulare Schicht des Celluloseethers aufgebracht, wobei dieser als Substituenten a) eine Trialkylsilylgruppe aufweist aber auch andere Substituenten aufweisen kann, die den Übertrag auf Oberflächen mit der Langmuir-Blodgett-(LB) und/oder LangmuirBlodgett-Schäfer-(LBS)-Technik ermöglichen.

Die Aufbringung dieser Schicht kann durch Inkubation in einer Lösung, durch einen Self-Assembly-(SA-)Prozeß oder vorzugsweise mit der Langmuir-Blodgett- oder Langmuir-Blodgett-Schäfer-Technik erfolgen. Die erfindungsgemäßen Celluloseethersind geeignet, sowohl auf hydrophilen wie auf hydrophoben Oberflächen zu haften. Somit kann diese Substanzklasse als oberflächenmodifizierender Film aufgetragen und verwendet werden.

Eine zusätzliche Stabilisierung erfahren diese Schichten, wenn photopolymerisierbare oder thermisch polymerisierbare Gruppen in das/die Molekül(e) eingebaut werden, z.B. Cinnamoylgruppen, aber auch alle anderen in der Chemie bekannten Gruppen, da diese durch die Polymerisation vor, während und nach der Übertragung die Schicht vernetzend stabilisieren (die eingangs genannten Druckschriften werden hier ausdrücklich mit ihrem Offenbarungsgehalt einbezogen).

Hierbei können die polymerisierbaren Gruppen entweder am obengenannten Celluloseether angebracht sein oder aber in Form eines weiteren Moleküls, das vermischt mit dem Celluloseether auf oder in die Schicht aufgebracht. wird, vorliegen. Die Polymerisation kann innerhalb einer Monolage stattfinden; sind jedoch mehrere Monolagen übereinander vorhanden, kann die Polymerisation auch zwischen Molekülen der einzelnen Schichten stattfinden.

Die erfindungsgemäßen Celluloseether finden verschiedene Anwendungen. So lassen sie sich als "Haftvermittler" einsetzen, die sich zwischen der Oberfläche des Trägermaterials und einer oder mehreren weiteren Schichten befinden. Weitere Schichten können mit allen bekannten Methoden aufgetragen werden, vorzugsweise jedoch mit Langmuir-Blodgett- oder Langmuir-Blodgett-Schäfer-Technik. Als weitere Schichten eignen sich besonders nicht-amphiphile Haar-Stab-Polymere, besonders polysaccharidhaltige Moleküle, wie Trialkylsilylcellulose selbst, z.B. Trimethylsilylcellulosecinnamoat, aber auch andere Derivate.

Eine besonders wichtige Anwendung dieser Mehrkomponentenschichten ist der Einsatz zur Immobilisierung von Molekülen an diesen Oberflächen. Hierbei besitzt mindestens die obere der auf der als "Haftvermittler" wirkenden ersten Schicht aufgebrachten Schichten funktionelle Gruppen in der Weise, daß andere Moleküle kovalent gebunden werden können. Hierzu zählen z.B. Aminogruppen, Aldehydgruppen, Carbonsäurederivate und/oder Gruppen, die sich in aktive Gruppen überführen lassen, z.B. olefinische Doppelbindungen, Zirntsäurederivate u.a.

Darüber hinaus eignet sich die Substanzklasse auch zur direkten Ankopplung von Molekülen aufgrund der vorhandenen Aminoalkylgruppen. Die Aminoalkylgruppen dienen als nukleophiles Agens und bilden kovalente Bindungen mit elektrophile Gruppen tragenden Molekülen.

Durch die vorhandenen Triarylsilylgruppen können die Oberflächeneigenschaften in der Weise verändert werden, daß die Silylgruppen nach der Beschichtung abgespalten werden, so daß Hydroxygruppen verbleiben. Dies kann z.B. durch die Einwirkung von Säure erreicht werden.

In jeweils bevorzugten Ausführungsfonnen des erfindungsgemäßen Verfahrens zusammengefaßt
- wird der Celluloseether vor, während oder nach der Durchführung der Beschichtung vernetzt,
- wird die Beschichtung als ganzes oder in Einzelschichten durch Zugabe eines Vernetzungsmittels vernetzt,
- besteht die Beschichtung aus einer oder mehreren Einzelschichten,
- ist in allen Schichten außerhalb des flächigen Trägermaterials Celluloseether vorhanden,
- stellt die Celluloseether enthaltende Schicht die alleinige Beschichtung dar,
- stellt die Celluloseether enthaltende Schicht eine Zwischenschicht zwischen flächigem Trägermaterial und Beschichtung dar, wobei die Beschichtung gegebenenfalls ebenfalls Celluloseether enthält,
- erfolgt die Anbindung von Biomolekülen am Celluloseether und
- wird der Substituent b) des Celluloseethers nach Abschluß der Beschichtung unter Rückbildung von OH-Gruppen abgespalten.

Ganz besonders bevorzugt ist erfindungsgemäß ein Verfahren zur Immobilisierung von Biomolekülen auf einem Trägermaterial, bei dem die Biomoleküle an oder in der Beschichtung gebunden werden und die Beschichtung durch LB-, LBS- oder SA-Technik erfolgt und photochemisch vernetzbare und nicht-amphiphile Moleküle enthält, bei dem das flächige Trägermaterial innerhalb oder außerhalb der Beschichtung mindestens einen gemischten Celluloseether gemäß der vorstehenden Beschreibung enthält.

Die Herstellung der erfindungsgemäßen gemischten Celluloseether kann so erfolgen, daß in einer Lösung von Trialkylsilylcellulose in einem organischen Lösemittel ein in diesem Lösemittel kaum oder unlösliches reaktives Aminoalkanderivat zugesetzt, die Umsetzung durchgeführt und das Endprodukt isoliert wird.

### Beispiele

### Beispiel 1: Synthese von Aminopropyl-trimethylsilylcellulose

Es wird 1 g Trimethylsilylcellulose (Tmsc) in einer Mischung aus 50 ml Tetrahydrofuran (THF) und 6 ml Pyridin gelöst. Zu dieser Lösung wird 2 g festes 1-Amino-3-brompropan gegeben. Durch die schlechte Löslichkeit des 1-Amino-3-brompropans in THF wird seine Polymerisation unterdrückt, da der gelöste Teil sofort abreagiert. Nach 20 h Reaktionszeit bei Raumtemperatur wird das Produkt abgesaugt, mit Methanol und Wasser gewaschen und umkristallisiert. Das Cellulosederivat wird in Chloroform gelöst (1µg/1µl) und auf einer Wasseroberfläche gespreitet. Schließlich wird es bei einem Oberflächendruck von 25 mN/m auf einen flächigen Glasträger übertragen.

### Beispiel 2: Synthese von Aminohexyl-trimethylsilylcellulose

3,28 g 6-Aminohexansäure werden in 40 ml einer 10 gew.-%igen wäßrigen Na₂CO₃-Lösung und 20 ml Dioxan gelöst. Bei 0°C wird eine Lösung von 6,47 g des 9-Fluorenylmethyl-chlorformiats (zum Schutz der Aminosäure) in 45 ml Dioxan während 15 min. unter Rühren zugegeben. Das Reaktionsprodukt wird in 600 ml H₂O gegeben und 2 x mit je 100 ml Diethylether ausgeschüttelt. Die wäßrige Phase wird mit HCl versetzt bis das Gemisch leicht sauer reagiert, danach mit je 200 ml Ethylacetat ausgeschüttelt, mit H₂O die organische Phase gereinigt, mit MgSO₄ getrocknet und das Lösemittel entfernt.

1,04 g 4-Dimethylaminopyridin und 0,5 g Trimethylsilylcellulose werden in 50 ml Methylenchlorid gelöst. Eine Suspension der - wie vorstehend dargestellt - geschützten Aminosäure in 40 ml Methylenchlorid wird zügig zugegeben und 1,75 g Dicyclohexylcarbodiimid in 10 ml Methylenchlorid zugetropft. Nach 2 h bei Raumtemperatur unter Rühren wird das Reaktionsgemisch eingeengt und das Endprodukt mit 200 ml Methanol ausgefüllt, mit Methanol gewaschen und aus Methylenchlorid umkristallisiert. Die Ausbeute beträgt 89 %.

Das so erhaltene Produkt kann - ähnlich wie in Beispiel 1 ausgeführt - in Rampentechnik, d.h. hydrodynamischer Strömung an einer Oberfläche, oder durch Herausziehen eines Substrates (z.B. Glas) bei Konstanthaltung des Oberflächdrucks eingesetzt werden. Bevorzugt wird auf einer so erzeugten Monoschicht ("Haftvermittler") die eigentliche Beschichtung - so wie vorstehend ausführlich dargestellt und mit Zitaten belegt - aufgebracht. Eine Immobilisierung von Biomolekülen (d.h. Moleküle wie DNS, Antikörper, Antigene, Enzyme, Hormone oder andere Peptide) kann erfolgreich durchgeführt werden.

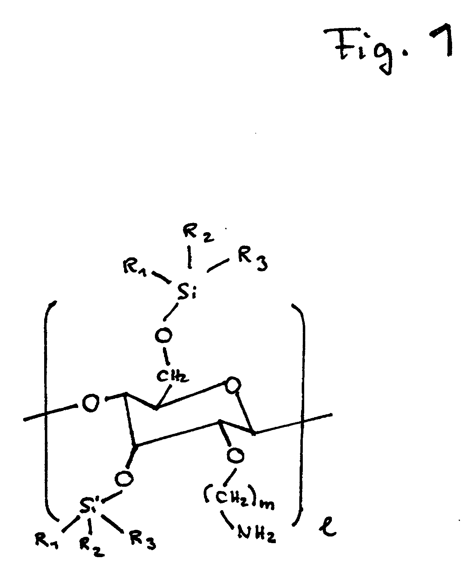

Die anschließende Strukturformel soll beispielhaft die erfindungsgemäßen Produkte erläutern, dabei bedeuten in Fig. 1 in der dort dargestellten vollständig an einer Anhydroglucose-Einheit substituierten (Mono-)Aminoalkyl(di-)trialkylsilylcellulose
m ≥ 1 (bevorzugt 2 bis 8)
R¹, R², R³ gleich oder verschieden bevorzugt CH₃ oder C₂H₅ 1 im Mittel ≥5

## Patentansprüche

1. Polysaccharidderivat enthaltend
a) mindestens einen hydrophoben und
b) mindestens einen ein Stickstoff enthaltenden Substituenten,
**dadurch gekennzeichnet, , daß** das Derivat ein Celluloseefher ist, der als Substituenten a) eine Trialkylsilyl- und als Substituenten b) eine Aminoalkylgruppe aufweist und einen Polymerisationsgrad von ≥5 hat.

2. Polysaccharidderivat nach Anspruch 1, **dadurch gekennzeichnet, daß** der Alkylrest im Substituenten a) 1 oder 2 C-Atome und im Substituenten b) 2 bis 8 C-Atome aufweist.

3. Polysaccharidderivat nachAnspruch 1 oder 2, **dadurch gekennzeichnet, daß** es zusätzlich
c) mindestens einen weiteren Substituenten enthält, der eine photochemisch, radikalisch oder thermisch vernetzbare Gruppe trägt.

4. Verfahren zur Immobilisierung von Biomolekülen auf einem beschichteten flächigen Trägermaterial, bei dem die Biomoleküle an oder in der Beschichtung gebunden werden, **dadurch gekennzeichnet, daß** das beschichtete flächige Trägermaterial innerhalb oder außerhalb der Beschichtung mindestens ein Polysaccharidderivat nach einem der Ansprüche 1 bis 3 enthält.

5. Verfahren nach Anspruch 4, **dadurch gekennzeichnet, daß** das Polysaccharidderivat vor, während oder nach der Durchführung der Beschichtung vernetzt wird.

6. Verfahren nach Anspruch 4, **dadurch gekennzeichnet, daß** die Beschichtung durch Zugabe eines Vernetzungsmittels vernetzt wird.

7. Verfahren nach einem der Ansprüche 4 bis 6, **dadurch gekennzeichnet, daß** die Beschichtung aus einer oder mehreren Einzelschichten besteht.

8. Verfahren nach einem der Ansprüche 4 bis 7, **dadurch gekennzeichnet, daß** in allen Schichten außerhalb des flächigen Trägermaterials Polysaccharidderivat vorhanden ist.

9. Verfahren nach einem der Ansprüche 4 bis 7, **dadurch gekennzeichnet, daß** die Polysaccharidderivat enthaltende Schicht die alleinige Beschichtung darstellt.

10. Verfahren nach einem der Ansprüche 4 bis 7, **dadurch gekennzeichnet, daß** die Polysaccharidderivat enthaltende Schicht eine Zwischenschicht zwischen flächigem Trägermaterial und Beschichtung darstellt, wobei die Beschichtung gegebenenfalls ebenfalls Polysaccharidderivat enthält.

11. Verfahren nach einem der Ansprüche 4 bis 10, **dadurch gekennzeichnet, daß** die Anbindung von Biomolekülen am Polysaccharidderivat erfolgt

12. Verfahren each einem der Ansprüche 4 bis 11, **dadurch gekennzeichnet, daß** der Substituent b) des Polysaccharidderivates nach Abschluß der Beschichtung unter Rückbildung von OH-Gruppen abgespalten wird.

13. Verfahren zur Immobilisierung von Biomolekülen auf einem Trägermaterial, bei dem die Biomoleküle an oder in der Beschichtung gebunden werden und die Beschichtung durch LB-, LBS- oder SA-Technik erfolgt und photochemisch vernetzbare und nicht-amphiphile Moleküle enthält, **dadurch gekennzeichnet, daß** das flächige Trägermaterial innerhalb oder außerhalb der Beschichtung mindestens einen gemischten Celluloseether nach einem der Ansprüche 1 bis 3 enthält.

14. Verfahren zur Herstellung des gemischten Celluloseethers nach einem der Ansprüche 1 bis 3, **dadurch gekennzeichnet, daß** in einer Lösung von Trialkylsilylcellulose in einem organischen Lösemittel ein in diesem Lösemittel kaum oder unlösliches reaktives Aminoalkanderivat zugesetzt, die Umsetzung durchgeführt und das Endprodukt isoliert wird.

## Claims

1. Polysaccharide derivative containing
a) at least one a hydrophobe-containing and
b) at least one nitrogen-containing substitute,
**characterized in that** the derivative is a cellulose ether, with as substituent a) a trialkyl silyl- and as substituent b) an aminoalkyl group and which has a degree of polymerization of ≥ 5.

2. Polysaccharide derivative according to claim 1, **characterized in that** the substituent a) has 1 or 2 C atoms and the substituent b) has 2 to 8 C atoms.

3. Polysaccharide derivative according to claim 1 or 2, **characterized in that** it also
c) contains at least one further substituent carrying a photochemically, radically or thermally cross-linkable group.

4. A method for the immobilisation of biomolecules on a coated flat support material, in which the biomolecules are bound on or in the coating, **characterized in that** the coated flat support material within or outside the coating contains at least one polysaccharide derivative according to claims 1 to 3.

5. A method according to claim 4, **characterized in that** the polysaccharide derivative us cross-linked before, during or after the coating process.

6. A method according to claim 4, **characterized in that** the coating is cross-linked by the addition of a cross-linking agent.

7. A method according to claims 4 to 6, **characterized in that** the coating consists of one or more individual layers.

8. A method according to one of claims 4 to 7, **characterized in that** a polysaccharide derivative is present in all layers except the flat support material.

9. A method according to one of claims 4 to 7, **characterized in that** the layer containing the polysaccharide derivative represents the only coating.

10. A method according to one of claims 4 to 7, **characterized in that** the layer containing the polysaccharide derivative represents a layer between the flat support material and the coating, wherein if appropriate the coating also contains a polysaccharide derivative.

11. A method according to one of claims 4 to 10, **characterized in that** the biomolecules are bound to the polysaccharide derivative.

12. A method according to one of claims 4 to 11, **characterized in that** the substituent b) of the polysaccharide derivative is eliminated after completion of the coating with the formation of OH groups.

13. A method of immobilisation of biological molecules on a support material, in which the biomolecules are bound on or in the coating and the coating process takes place by the LB, LBS or SA technique and contains photo-chemically cross-linkable and non-amphiphilic molecules, **characterized in that** the flat support material contains at least one mixed cellulose ether within or outside the coating according to one of claims 1 to 3.

14. A method for the production of mixed cellulose ethers according to one of claims 1 to 3, **characterized in that** in a solution of trialkyl silyl cellulose in an organic solvent, a poorly soluble or insoluble reactive aminoalkane derivative is added, the reaction carried out and the end product isolated.

## Revendications

1. Dérivé de polysaccharide contenant
a) au moins un substituant hydrophobe, et
b) au moins un substituant contenant de l'azote,
**caractérisé en ce que** le dérivé est un éther de cellulose, qui en tant que substituant a) possède un groupe trialkylsilyle et en tant que substituant b) un groupe aminoalkyle, et a un degré de polymérisation ≥ 5.

2. Dérivé de polysaccharide selon la revendication 1, **caractérisé en ce que** le radical alkyle comporte an le substituant a) 1 ou 2 atomes de carbone et dans le substituant b) de 2 à 8 atomes de carbone.

3. Dérivé de polysaccharide selon la revendication 1 ou 2, **caractérisé en ce qu'**il contient en outre
c) au moins un autre substituant, qui porte un groupe réticulable par voie photochimique, radicalaire ou thermique.

4. Procédé pour immobiliser des biomolécules sur un matériau support bidimensionnel revêtu, dans lequel les biomolécules sont liées au revêtement ou à l'intérieur de ce dernier, **caractérisé en ce que** le matériau support bidimensionnel revêtu contient, à l'intérieur ou à l'extérieur du revêtement, au moins un dérivé de polysaccharide selon l'une des revendications 1 à 3.

5. Procédé selon la revendication 4, **caractérisé en ce que** le dérivé de polysaccharide est réticulé avant, pendant ou après mise en oeuvre du revêtement.

6. Procédé selon la revendication 4, **caractérisé en ce que** le revêtement est réticulé par addition d'un agent de réticulation.

7. Procédé selon l'une des revendications 4 à 6, **caractérisé en ce que** le revêtement est constitué d'une ou plusieurs monocouches.

8. Procédé selon l'une des revendications 4 à 7, **caractérisé en ce qu'**un dérivé de polysaccharide est présent dans toutes les couches à l'extérieur du matériau support bidimensionnel.

9. Procédé selon l'une des revendications 4 à 7, **caractérisé en ce que** la couche contenant un dérivé de polysaccharide représente le revêtement unique.

10. Procédé selon l'une des revendications 4 à 7, **caractérisé en ce que** la couche contenant le dérivé de polysaccharide représente une couche intermédiaire entre le matériau support bidimensionnel et le revêtement, le revêtement contenant éventuellement lui aussi un dérivé de polysaccharide.

11. Procédé selon l'une des revendications 4 à 10, **caractérisé en ce que** la liaison des molécules s'effectue sur le dérivé de polysaccharide.

12. Procédé selon l'une des revendications 4 à 11, **caractérisé en ce que** le substituant b) du dérivé de polysaccharide est dissocié après la fin du revêtement, avec formation en retour de groupes OH.

13. Procédé pour immobiliser des biomolécules sur un matériau support, dans lequel les biomolécules sont liées au revêtement ou à l'intérieur de ce dernier, et le revêtement est réalisé par la technique LB, LBS ou SA, et contient des molécules non-amphiphiles et réticulables par voie photochimique, **caractérisé en ce que** le matériau support bidimensionnel contient, à l'intérieur ou à l'extérieur du revêtement, au moins un éther mixte de cellulose selon l'une des revendications 1 à 3.

14. Procédé de préparation de l'éther mixte de cellulose selon l'une des revendications 1 à 3, **caractérisé en ce qu'**on ajoute, dans une solution de trialkylsilylcellulose dans un solvant organique, un dérivé d'aminoalcanes à peine soluble ou insoluble dans ce solvant, on met en oeuvre la réaction et on isole le produit final.
